# EUROPEAN PATENT APPLICATION

(11) **EP 4 109 097 A1**
(43) Date of publication of application: **28.12.2022**
(21) Application number: 21757574.5
(22) Date of filing: 08.02.2021
(51) Int. Cl.: G01N 33/53, C12Q 1/06, G01N 33/543

(54) **METHOD FOR ANALYZING BIOLOGICAL SAMPLE**

(30) Priority: 17.02.2020 JP 2020024506
(71) Applicant: JVCKenwood Corporation, Yokohama-shi, Kanagawa 2210022 (JP)
(72) Inventor: TSUJITA Koji, Yokohama-shi, Kanagawa 221-0022 (JP); ONO Masayuki, Yokohama-shi, Kanagawa 221-0022 (JP); ITONAGA Makoto, Yokohama-shi, Kanagawa 221-0022 (JP); HASEGAWA Yuichi, Yokohama-shi, Kanagawa 221-0022 (JP); OHSHIMA Katsunori, Yokohama-shi, Kanagawa 221-0022 (JP); SAITOU Atsushi, Yokohama-shi, Kanagawa 221-0022 (JP); KAWAKAMI Tatsuya, Yokohama-shi, Kanagawa 221-0022 (JP)
(74) Representative: Carstens, Dirk Wilhelm
(86) International application number: PCT/JP2021/004569
(87) International publication number: WO 2021/166715

(57) **Abstract**

A first buffer fluid containing a first bead having fixed to a surface thereof a first antibody that specifically binds to a first antigen is injected into a reaction container, and then a plurality of a second bead having fixed to a surface thereof a second antibody that specifically binds to an optional second antigen, and a biological sample containing an exosome to be analyzed having the first antigen and the second antigen on a surface of the exosome are injected into the reaction container, thereby generating a second buffer fluid (S1 to S3). An exosome having the first bead and the second bead bound thereto is collected from the second buffer fluid (S4). The exosome having the first bead and the second bead bound thereto is separated into the first bead, the second bead, and the exosome, and the second bead and the exosome are individually collected (S5 and S6). An inclusion of the exosome is analyzed (S7), and the number of second beads is counted (S8).

## Description

### [TECHNICAL FIELD]

The present disclosure relates to a biological sample analysis method of analyzing inclusions of exosomes contained in a biological sample, and counting the number of exosomes.

### [BACKGROUND ART]

Antigens associated with specific diseases are detected and analyzed as biomarkers to detect specific diseases or to verify the efficacy of treatments for specific diseases. Exosomes, which are secreted from cells and contained in various body fluids such as blood, are promising as biomarkers for detecting specific diseases.

Exosomes are minute membrane vesicles covered with a lipid bilayer, which contains various membrane proteins such as CD9 and CD63. It is known that when a person suffers from a specific disease, there is an increase in the number of exosomes in which disease-related membrane proteins are expressed on the surface of the lipid bilayer. By counting the number of exosomes with disease-related antigens using disease-related membrane proteins expressed on the surface of exosomes as antigens, it is possible to detect whether a person has a specific disease or not.

Inside exosomes, there are nucleic acids such as miRNA (microRNA) and DNA, and inclusions such as proteins. Inclusions of exosomes are also promising as biomarkers for the detection of specific diseases (see Patent Literature 1) .

### [CITATION LIST]

### [PATENT LITERATURE]

Patent Literature 1: Japanese Unexamined Patent Application Publication No. 2018-163043
Patent Literature 2: Japanese Unexamined Patent Application Publication No. 2017-40595
Patent Literature 3: Japanese Unexamined Patent Application Publication No. 2019-211366

### [SUMMARY OF INVENTION]

Conventionally, two analytes for a biological sample containing exosomes are prepared, and the inclusions of exosomes are analyzed in one sample, and the number of exosomes are counted in the other sample. In other words, the exosomes in which the inclusions are analyzed are not the same exosomes in which the number thereof is counted. It is desirable to analyze inclusions of exosomes and count the number of exosomes using one analyte.

One or more embodiments aim to provide a biological sample analysis method capable of analyzing inclusions of exosomes and counting the number of exosomes using only one biological sample.

An aspect of one or more embodiments provides a biological sample analysis method including: injecting, into a reaction container, a first buffer fluid containing a plurality of a first bead having fixed to a surface thereof a first antibody that specifically binds to a first antigen associated with a particular disease; injecting, into the reaction container, a plurality of a second bead having fixed to a surface thereof a second antibody that specifically binds to an optional second antigen that is the same as or different from the first antigen, and a biological sample containing an exosome to be analyzed having the first antigen and the second antigen on a surface of the exosome, thereby generating a second buffer fluid; collecting the exosome to be analyzed having the first bead and the second bead bound thereto, from the second buffer fluid; separating the collected exosome to be analyzed having the first bead and the second bead bound thereto, into the first bead, the second bead, and the exosome to be analyzed, and collecting the second bead and the exosome to be analyzed; and analyzing an inclusion of the collected exosome to be analyzed, and counting the number of collected second beads.

The biological sample analysis method according to one or more embodiments makes it possible to analyze inclusions of exosomes and count the number of exosomes using only one biological sample.

### [BRIEF DESCRIPTION OF DRAWINGS]

FIG. 1 is a flowchart illustrating a biological sample analysis method according to one or more embodiments.
FIG. 2 is a conceptual diagram illustrating a magnetic microbead for use in the biological sample analysis method according to one or more embodiments.
FIG. 3 is a conceptual diagram illustrating a reaction container injected with buffer fluid containing magnetic microbeads.
FIG. 4 is a conceptual diagram illustrating an analyte of a biological sample containing exosomes.
FIG. 5 is a conceptual diagram illustrating the structure of an exosome.
FIG. 6 is a conceptual diagram illustrating antibody nanobeads for use in the biological sample analysis method according to one or more embodiments.
FIG. 7 is a conceptual diagram illustrating an analyte containing exosomes and antibody nanobeads injected into a reaction container injected with buffer fluid containing a magnetic microbead, and the exosomes sandwiched between the magnetic microbead and the antibody nanobeads.
FIG. 8 is a conceptual diagram of the reaction container illustrated in FIG. 7 after removal of unwanted materials other than the magnetic microbead and the antibody nanobeads which sandwich the exosomes.
FIG. 9 is a diagram illustrating a method of separating the unwanted materials from the reaction container illustrated in FIG. 7.
FIG. 10 is a conceptual diagram illustrating a state where the antibody nanobeads from among the magnetic microbead and the antibody nanobeads which sandwiched the exosomes are detached from the exosomes.
FIG. 11 is a conceptual diagram illustrating the magnetic microbead obtained by detaching the magnetic microbead and the antibody nanobeads which captured the exosomes.
FIG. 12 is a conceptual diagram illustrating the antibody nanobeads obtained by detaching the magnetic microbead and the antibody nanobeads which captured the exosomes.
FIG. 13 is a diagram illustrating a method of detaching the antibody nanobeads from the exosomes using a surfactant.
FIG. 14 is a diagram illustrating a method of detaching the antibody nanobeads from the exosomes using a hapten.
FIG. 15 is a diagram illustrating a method of detaching the antibody nanobeads from the exosomes using a linker.
FIG. 16A is a diagram illustrating the steps up to the middle of the method of separating the magnetic microbeads and the antibody nanobeads.
FIG. 16B is a diagram illustrating the steps following those in FIG. 16A of the method of separating the magnetic microbeads and the antibody nanobeads.
FIG. 17 is a flowchart illustrating a specific method of counting the number of antibody nanobeads in step S8 of FIG. 1.
FIG. 18 is a diagram illustrating the method of counting the number of antibody nanobeads.
FIG. 19 is a conceptual diagram illustrating the formation of polar functional groups for attaching the antibody nanobeads to a disk substrate.

### [DESCRIPTION OF EMBODIMENTS]

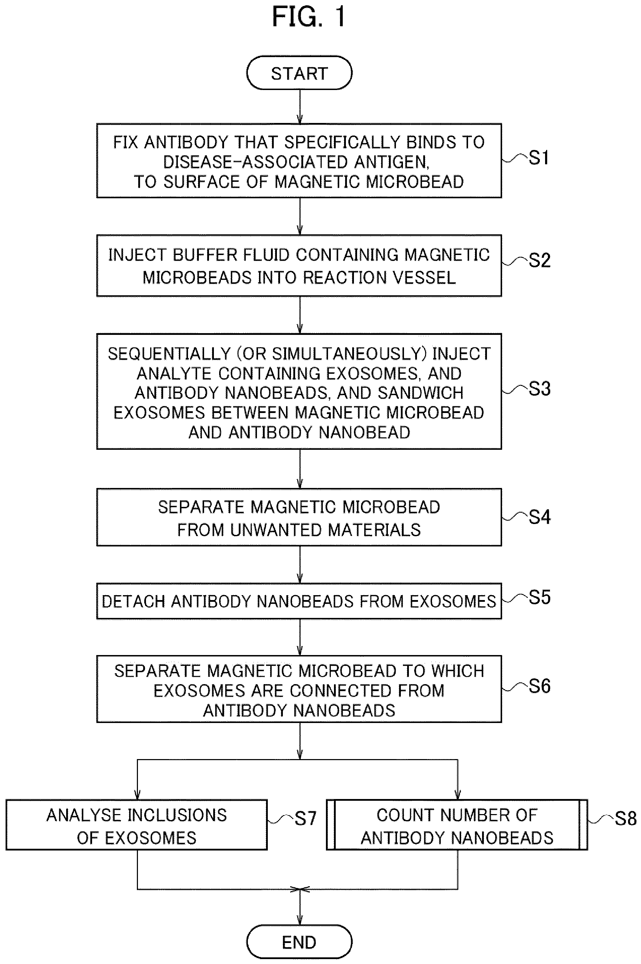

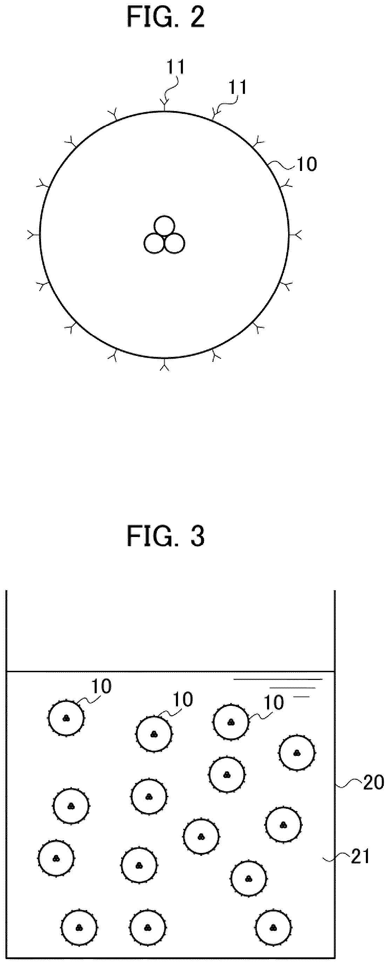

Hereinafter, a biological sample analysis method according to one or more embodiments will be described with reference to the accompanying drawings. In FIG. 1, an operator, in step S1, fixes a plurality of antibodies 11 that specifically bind to disease-associated antigens to the surface of a magnetic microbead 10, as illustrated in FIG. 2. As illustrated in FIG. 3, in step S2, the operator injects buffer fluid 21 (first buffer fluid) containing a number of magnetic microbeads 10 to which the antibodies 11 are fixed into a reaction container 20. The diameters of the magnetic microbeads 10 may be about 3 µm, and the diameters are preferably on the order of micrometers.

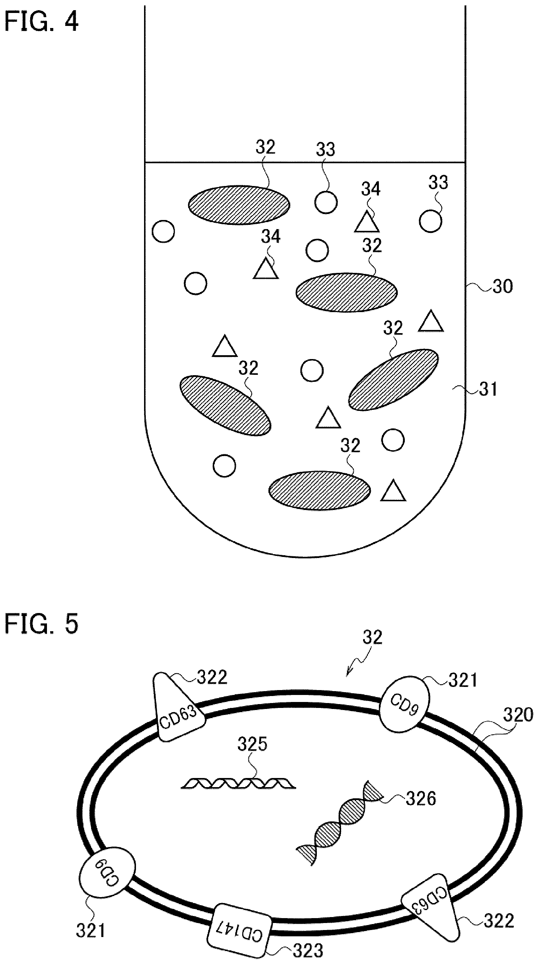

As illustrated in FIG. 4, the operator prepares an analyte 31 of a biological sample containing exosomes 32 in a container 30. The analyte 31 is optional fluid such as blood, and contains contaminants 33 and 34 which are unwanted materials in addition to the exosomes 32. As illustrated conceptually in FIG. 5, the exosome 32 is covered with a lipid double membrane 320, and the lipid double membrane 320 contains, for example, membrane proteins 321 to 323. The membrane protein 321 is CD9, the membrane protein 322 is CD63, and the membrane protein 323 is CD147. CD147 is an example of a disease-associated membrane protein, and CD9 and CD63 are membrane proteins present in both normal exosomes and exosomes with disease-associated antigens.

In one or more embodiments, CD147 is an antigen (first antigen) associated with a particular disease. That is, the antibody 11 fixed to the surface of the magnetic microbead 10 is an antibody (first antibody) that specifically binds to CD147. The exosome 32 in which CD147 exists is used as an exosome to be analyzed.

Inside the exosome 32 are nucleic acids such as miRNA 325 and DNA 326, and unillustrated inclusions such as proteins.

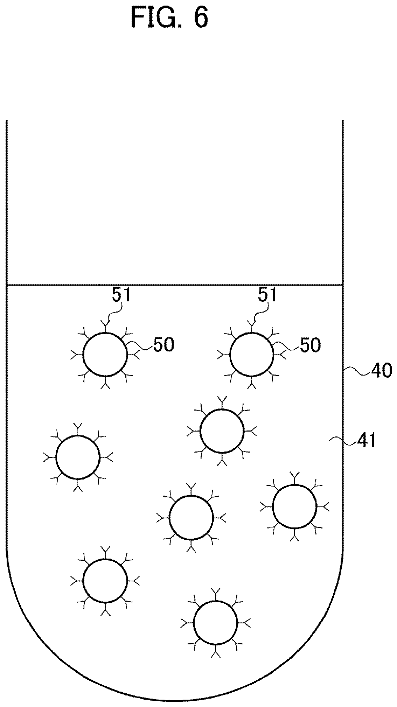

As illustrated in FIG. 6, the operator prepares in the container 40, buffer fluid 41 containing antibody nanobeads 50 having a plurality of antibodies 51 fixed to the surface thereof. The antibody nanobeads 50 are much smaller in diameter than the magnetic microbeads 10 and have a diameter of about 200 nm. The diameter of the antibody nanobeads 50 may be less than 1 µm, preferably from 100 nm to 500 nm. The antibody 51 fixed to the surface of the antibody nanobead 50 is an antibody (second antibody) that binds to any antigen (second antigen) present on the surface of the exosome 32 such as CD9, CD63 and CD147. That is, the second antigen may be the same as or different from the first antigen.

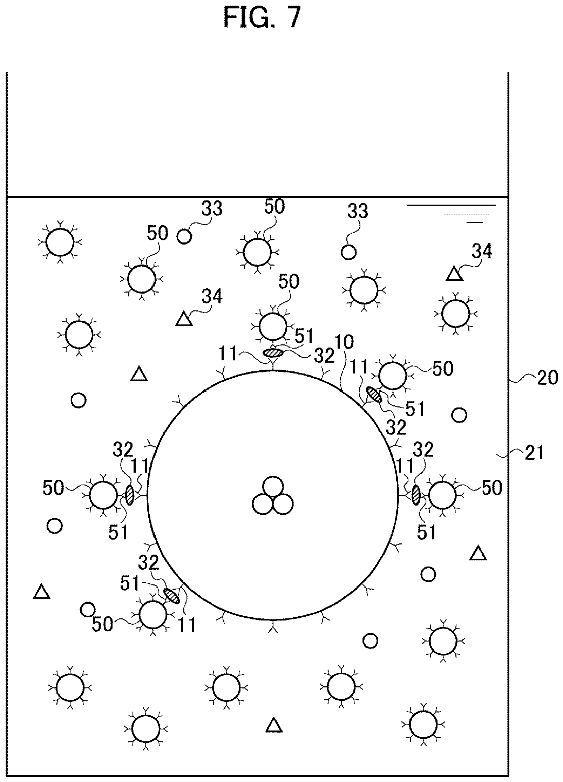

As illustrated in FIG. 7, the operator sequentially (or simultaneously) injects the analyte 31 containing the exosomes 32 and the buffer fluid 41 containing the antibody nanobeads 50 into the reaction container 20 in step S3. When the buffer fluid 21 is a mixture of the buffer fluid 21 and the buffer fluid 41, buffer fluid 21 (second buffer fluid) which contains the exosomes 32 sandwiched between the magnetic microbead 10 and the antibody nanobeads 50 is generated in the reaction container 20. In this case, since "sandwich" means that the two beads bind to one exosome, it is possible to say that second buffer fluid which contains the exosomes 32 having the magnetic microbead 10 and the antibody nanobeads 50 bound thereto is generated. At this time, the operator shakes the reaction container 20 as necessary and waits for a predetermined time. In FIG. 7, the exosomes 32 in which CD147 is present are sandwiched between the magnetic microbead 10 and the antibody nanobeads 50.

In FIG. 7, there are a number of magnetic microbeads 10 in the reaction container 20 in reality, but for ease of understanding, only an enlarged single magnetic microbead 10 is illustrated.

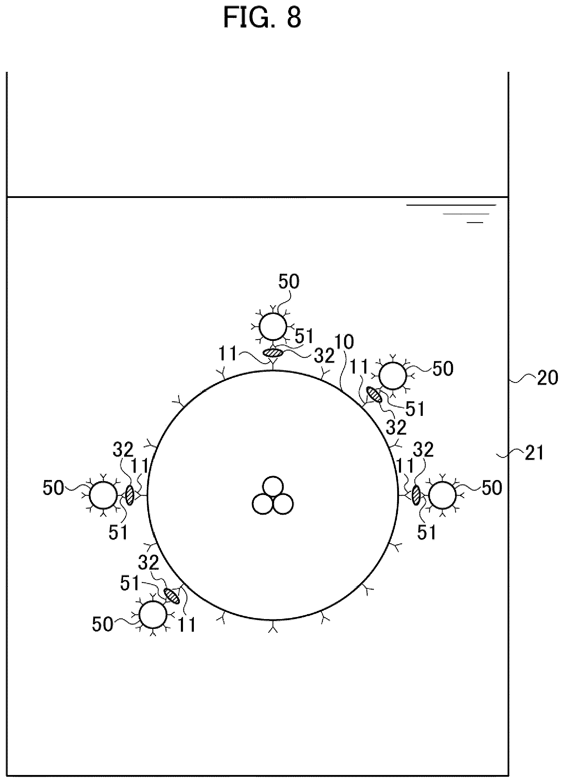

In step S4, the operator separates the magnetic microbead 10 to which the antibody nanobeads 50 are connected via the exosomes 32 from the unwanted materials by means of magnetic collection, weak centrifugation, or the like. The operator washes the separated magnetic microbead 10. The antibody nanobeads 50 that are not connected to the magnetic microbead 10 and the contaminants 33 and 34 are unwanted materials. Since the antibody nanobeads 50 are excessively injected in step S3, the unwanted materials are mainly the antibody nanobeads 50 not connected to the magnetic microbead 10. As a result of step S4, the magnetic microbead 10 to which the antibody nanobeads 50 are connected via the exosomes 32 can be collected, as illustrated in FIG. 8.

Meanwhile, in a case where the antibody nanobeads 50 are not magnetic nanobeads, the magnetic microbead 10 can be easily separated from the antibody nanobeads 50 which are not connected to the magnetic microbead 10 by magnetically collecting the magnetic microbead 10. When the number of the antibody nanobeads 50 connected to the magnetic microbead 10 is counted in step S8 described later, the antibody nanobeads 50 are preferably magnetic nanobeads. Even if the antibody nanobeads 50 are magnetic nanobeads, it is possible to separate the magnetic microbead 10 from the unconnected antibody nanobeads 50.

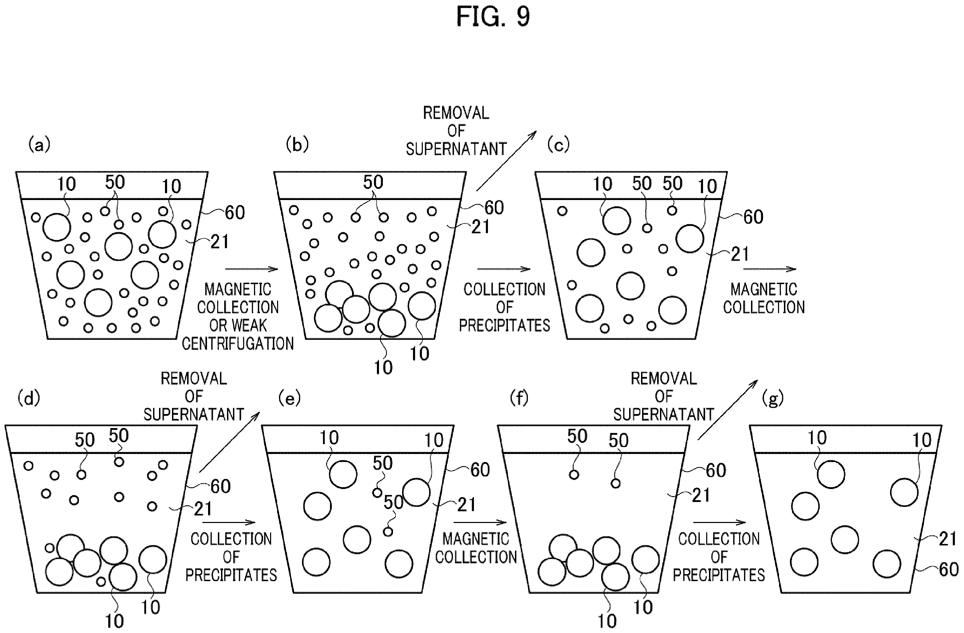

Specifically, as illustrated in FIG. 9, it is possible to separate magnetic microbeads 10 from unconnected antibody nanobeads 50. In FIG. 9, (a) illustrates a state where a container 60 is injected with the buffer fluid 21 which contains the magnetic microbeads 10 to which the antibody nanobeads 50 are connected via the exosomes 32 and the unconnected antibody nanobeads 50. Part (a) of FIG. 9 corresponds to the state of FIG. 7. Magnetic collection in which a magnet is placed at the bottom of the container 60 or weak centrifugation can mostly separate the magnetic microbeads 10 from the unconnected antibody nanobeads 50, as illustrated in (b) of FIG. 9.

This is because the volumes of the magnetic microbeads 10 and the antibody nanobeads 50 differ by about 1000 times, which means that the magnetic microbeads 10 are magnetically collected in a short time, while the antibody nanobeads 50 are magnetically collected only after a long time. By utilizing the time difference in this magnetic collection, the magnetic microbeads 10 and the unconnected antibody nanobeads 50 can be mostly separated.

In the step of washing the magnetic microbeads 10, when the precipitates, which are mainly the magnetic microbeads 10 in (b) of FIG. 9, are collected, the supernatant is removed, and the buffer fluid 21 is added, the number of unconnected antibody nanobeads 50 can be greatly reduced as illustrated in (c) of FIG. 9. When magnetic collection is performed in the state illustrated in (c) of FIG. 9, (d) of FIG. 9 is achieved, and when the precipitates are collected, the supernatant is removed, and the buffer fluid 21 is added, the unconnected antibody nanobeads 50 can be further greatly reduced as illustrated in (e) of FIG. 9.

When magnetic collection is performed in the state illustrated in (e) of FIG. 9, (f) of FIG. 9 is achieved, and when the precipitates are collected, the supernatant is removed, and the buffer fluid 21 is added, it is possible to separate only the magnetic microbeads 10 to which the antibody nanobeads 50 are connected via the exosomes 32 as illustrated in (g) of FIG. 9. Part (g) of FIG. 9 corresponds to the state of FIG. 8. The number of times the precipitates are collected and the supernatant is removed is not limited to the number of times illustrated in FIG. 9.

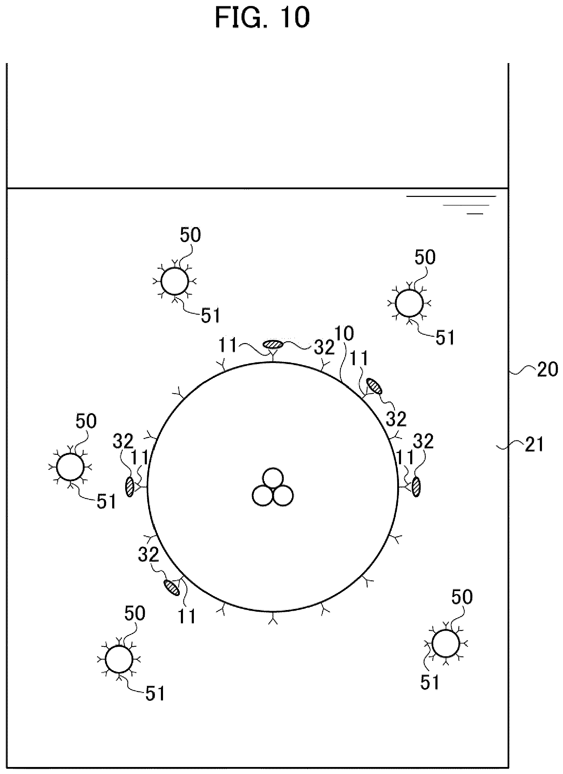

As illustrated in FIG. 10, in step S5, the antibody nanobeads 50 from among the magnetic microbead 10 and the antibody nanobeads 50 which sandwiched the exosomes 32 are detached from the exosomes 32 by the operator. In step S6, the operator separates the magnetic microbead 10 to which the exosomes 32 are connected from the antibody nanobeads 50.

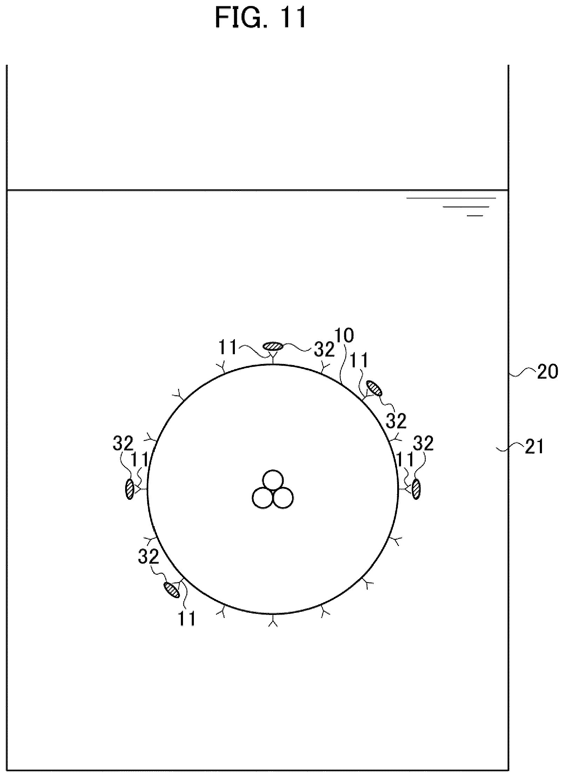

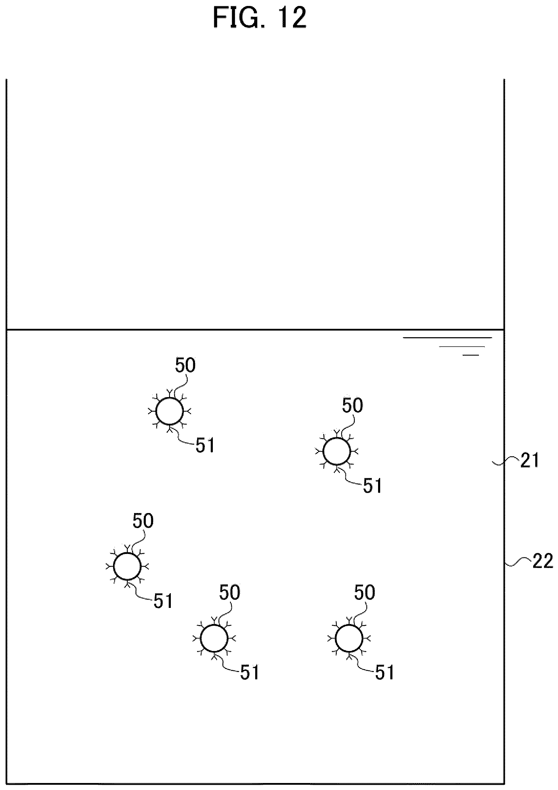

Thereafter, the magnetic microbead 10 having the exosomes 32 connected to the antibodies 11 illustrated in FIG. 11, and the antibody nanobeads 50 illustrated in FIG. 12 can be collected individually. In FIG. 11, buffer fluid 21 containing only the magnetic microbead 10 to which the exosomes 32 are connected is injected into the reaction container 20. In FIG. 12, buffer fluid 21 containing only the antibody nanobeads 50 is injected into the container 22.

One of the following methods can be employed as the process for detaching the antibody nanobeads 50 from the exosomes 32 in step S5. As a first method, a dissolution process can be employed in which the lipid double membrane 320 of the exosome 32 is broken by a surfactant in the state where the exosome 32 is sandwiched between the magnetic microbead 10 and the antibody nanobead 50.

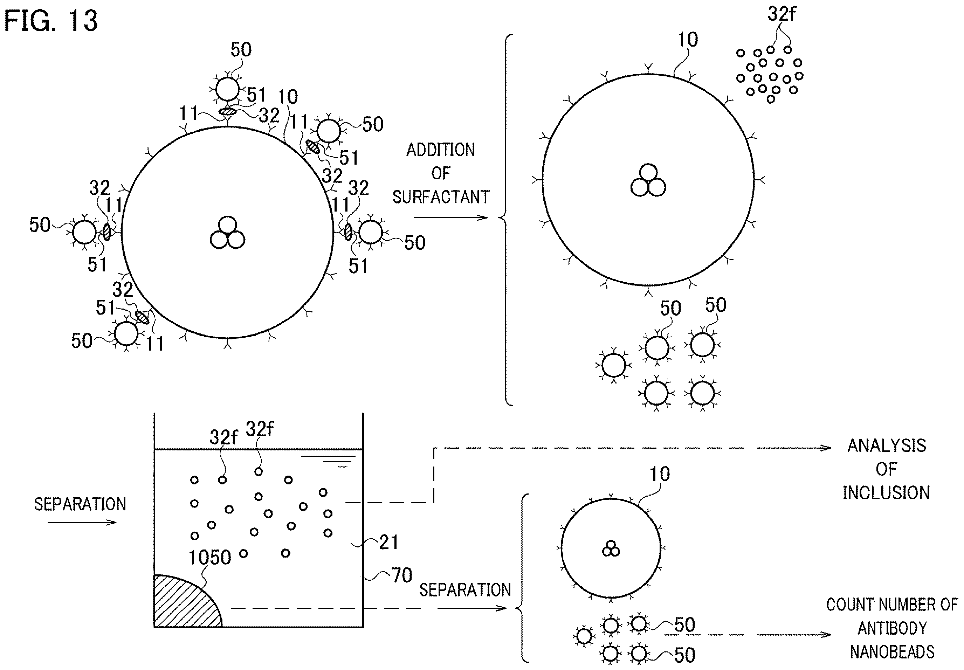

As illustrated in FIG. 13, for example, a solution of Triton X-100, which is a 2% nonionic surfactant, is added to the buffer fluid 21 in the state illustrated in FIG. 8, in which the exosomes 32 are sandwiched between the magnetic microbead 10 and the antibody nanobeads 50. As a result, the lipid double membrane 320 of the exosome 32 is broken and inclusions 32f are released into the buffer fluid 21. When a surfactant is added, the exosome 32 can be degraded in a shorter time by shaking and waiting for a predetermined time. Accordingly, the magnetic microbead 10, the antibody nanobeads 50, and the exosomes 32 (the inclusions 32f) are detached from each other.

When the buffer fluid 21, which contains the magnetic microbeads 10, the antibody nanobeads 50 and the inclusions 32f of the exosomes 32, is injected into a container 70 and centrifuged therein, an aggregate 1050 of the magnetic microbeads 10 and the antibody nanobeads 50 is obtained. By separating and washing the aggregate 1050, the magnetic microbeads 10 and the antibody nanobeads 50 can be collected individually.

In step S7, the operator removes the inclusions 32f of the exosomes 32 contained in the supernatant of the container 70. The operator analyzes the inclusions 32f of the exosomes 32. At this time, a DNA or protein in the inclusions 32f can be analyzed by an analytical method such as RT-PCR (reverse transcription-polymerase chain reaction) analysis or LC-MS (liquid chromatography-mass spectrometry) analysis. In step S8, the operator counts the number of antibody nanobeads 50. The operator may perform steps S7 and S8 in an optional order, or these steps may be performed simultaneously by a plurality of operators. When the operator has performed steps S7 and S8, the process of the biological sample analysis is completed.

Since the antibody nanobeads 50 that are counted in step S8 are bound to the exosomes 32, the number of the exosomes 32 in which CD147 is present and bound to the magnetic microbead 10 is counted in step S8. The exosomes 32 in which the inclusions 32f are analyzed in step S7 are bound to the antibody nanobeads 50 that are counted in step S8.

Thus, the exosomes 32 in which the inclusions 32f are analyzed are the same as the exosomes 32 that are counted. In one analyte 31 illustrated in FIG. 4, the inclusions 32f of the exosomes 32 can be analyzed and the number of exosomes 32 can be counted. Here, CD147, which is a membrane protein associated with a specific disease, is present in the exosomes 32.

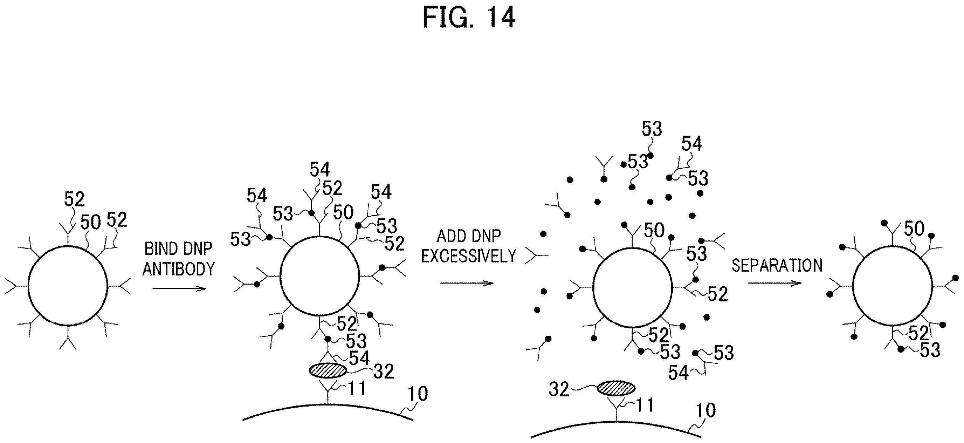

A second method of detaching the antibody nanobeads 50 from the exosomes 32 is to use a hapten illustrated in FIG. 14. A hapten is a substance that binds to an antibody but, because of its low molecular weight, does not exhibit any activity (immunogenicity) of inducing antibody production. A hapten becomes an immunogenic complete antigen when bound to an appropriate protein.

As illustrated in FIG. 14, the anti-DNP antibodies 52 are fixed to the antibody nanobead 50 in place of the antibodies 51. DNP is 2,4-dinitrophenol, which is an example of a hapten. The exosome 32 is bound to the anti-DNP antibody 52 via an antibody 54 (DNP antibody 54) to which the DNP 53 is bound. The DNP antibody 54 is an antibody that binds to any antigen present in the exosome 32 such as CD9, CD63, and CD147.

In this state, the binding of the anti-DNP antibody 52 to the DNP antibody 54 is an equilibrium reaction. When the DNP 53 (hapten) is excessively added to the buffer fluid 21 (third buffer fluid) containing the exosome sandwiched between the magnetic microbead 10 and the antibody nanobead 50, the DNP antibody 54 bound to the anti-DNP antibody 52 is replaced by the DNP 53, and the magnetic microbead 10 bound to the exosome 32 is detached from the antibody nanobead 50. When the magnetic microbead 10 to which the exosome 32 is bound and the antibody nanobead 50 are separated from each other and washed, the magnetic microbead 10 to which the exosome 32 is bound and the antibody nanobead 50 can be collected individually.

When the exosome 32 bound to the magnetic microbead 10 is dissolved in the same manner as in the first method, the inclusions 32f of the exosome 32 are analyzed in step S7. Further, in step S8, the number of the collected antibody nanobeads 50 is counted.

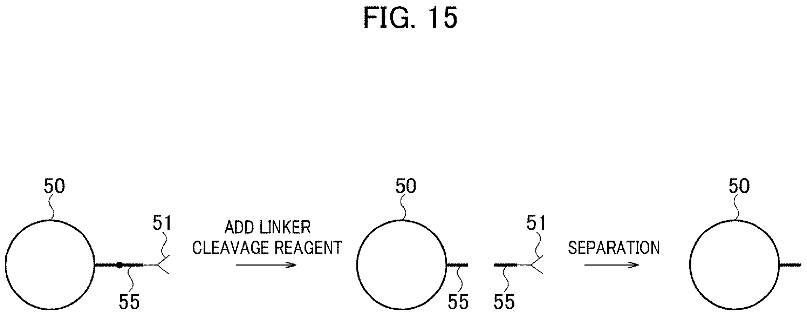

A third method of detaching the antibody nanobead 50 from the exosome 32 is to use a cleavable linker 55 illustrated in FIG. 15. The antibody 51 is fixed to the antibody nanobead 50 via the linker 55. In FIG. 15, only one antibody 51 is illustrated for simplicity. In a case where the third method is employed, the antibody 51 is fixed via the linker 55 to the antibody nanobead 50 illustrated in FIG. 8, and the antibody 51 is bound to the exosome 32.

When a linker cleavage reagent is added to the buffer fluid 21 (third buffer fluid) in the reaction container 20, the linker 55 is cleaved to detach the antibody nanobead 50 from the antibody 51 and to thereby detach the antibody nanobead 50 from the magnetic microbead 10 bound to the exosome 32, as illustrated in FIG. 15. When the antibody nanobead 50 and the magnetic microbead 10 bonded to the exosome 32 are separated from each other and washed, the antibody nanobead 50 and the magnetic microbead 10 bound to the exosome 32 can be collected individually.

When the exosome 32 bound to the magnetic microbead 10 is dissolved in the same manner as in the first method, the inclusions 32f of the exosome 32 are analyzed in step S7. Further, in step S8, the number of the collected antibody nanobeads 50 is counted.

As a fourth method of detaching the antibody nanobead 50 from the exosome 32, a Fab antibody may be used for the magnetic microbead 10 and a whole antibody may be used for the antibody nanobead 50. Degradation and digestion of the whole antibody using enzymes allows the antibody nanobead 50 to be detached from the exosome 32.

Separation of the magnetic microbeads 10 and the antibody nanobeads 50 in step S6 can be performed in the same manner as in FIG. 9. However, regarding the separation performed in step S6, it is necessary to collect the antibody nanobeads 50 contained in the supernatant without removing the supernatant.

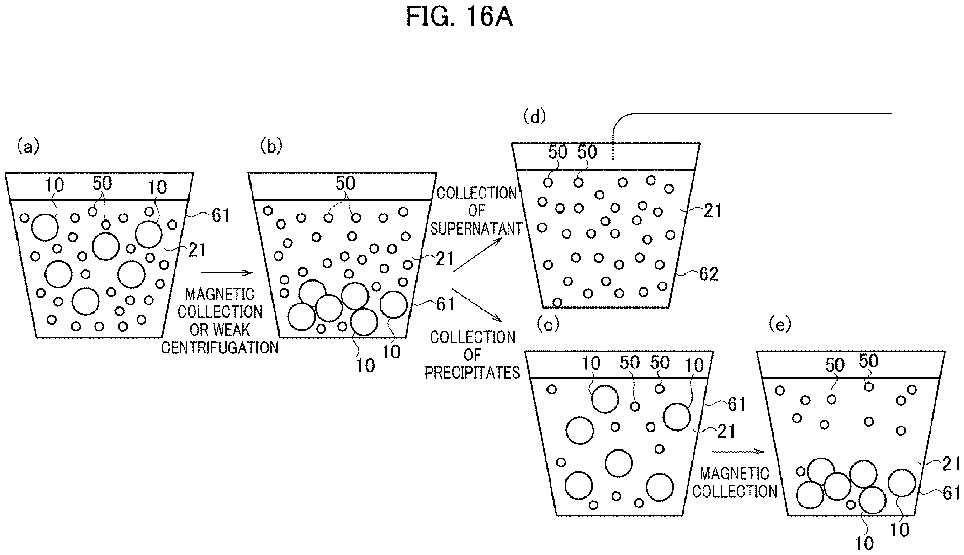

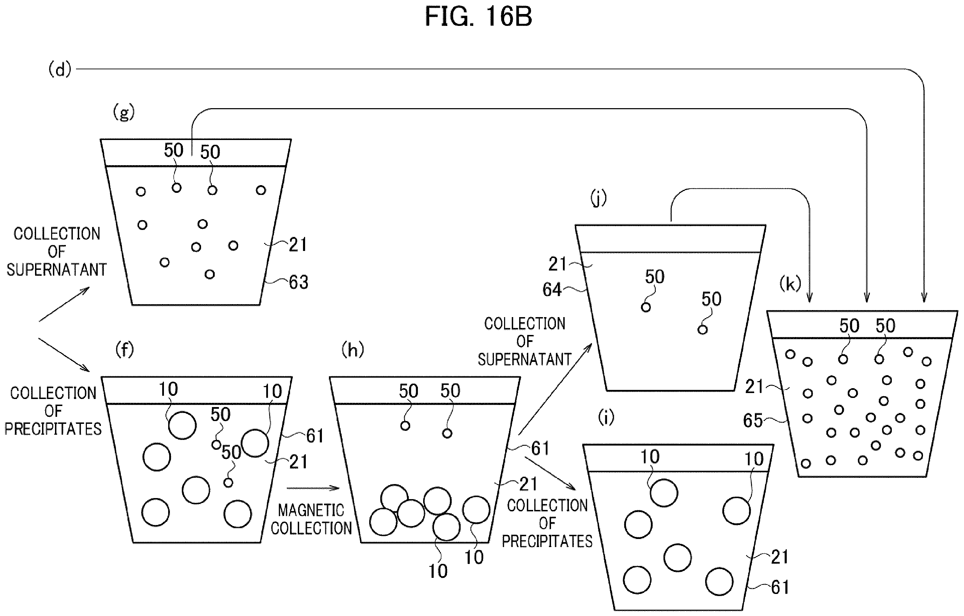

Specifically, as illustrated in FIGS. 16A and 16B, the magnetic microbeads 10 and the antibody nanobeads 50 can be separated. In FIG. 16A, (a) illustrates a state where a container 61 is injected with the buffer fluid 21 which contains the magnetic microbeads 10 and antibody nanobeads 50 separated from each other. Part (a) of FIG. 16A corresponds to the state of FIG. 10. Magnetic collection in which a magnet is placed at the bottom of the container 61 or weak centrifugation can mostly separate the magnetic microbeads 10 from the antibody nanobeads 50, as illustrated in (b) of FIG. 16A.

When the precipitates, which are mainly the magnetic microbeads 10 in (b) of FIG. 16A, are collected and the buffer fluid 21 is added, the antibody nanobeads 50 can be greatly reduced as illustrated in (c) of FIG. 16A. As illustrated in (d) of FIG. 16A, the supernatant is collected into the container 62 to obtain the buffer fluid 21 containing a large number of the antibody nanobeads 50. When magnetic collection is performed in the state illustrated in (c) of FIG. 16A, (e) of FIG. 16A is achieved.

When the precipitates, which are mainly the magnetic microbeads 10 in (e) of FIG. 16A, are collected and the buffer fluid 21 is added, the antibody nanobeads 50 can be further reduced as illustrated in (f) of FIG. 16B. As illustrated in (g) of FIG. 16B, the supernatant is collected into the container 63 to obtain the buffer fluid 21 containing the antibody nanobeads 50. When magnetic collection is performed in the state illustrated in (f) of FIG. 16B, (h) of FIG. 16B is achieved.

When the precipitates, which are only the magnetic microbeads 10 in (h) of FIG. 16B, are collected and the buffer fluid 21 is added, the buffer fluid 21 containing only the magnetic microbeads 10 and not containing the antibody nanobeads 50 can be obtained as illustrated in (i) of FIG. 16B. Part (i) of FIG. 16B corresponds to the state of FIG. 11. As illustrated in (j) of FIG 16B, the supernatant is collected into the container 64 to obtain the buffer fluid 21 containing a small number of the antibody nanobeads 50.

As illustrated in (k) of FIG. 16B, when the antibody nanobeads 50 in all of the containers 62 to 64 are combined, all the antibody nanobeads 50 which were bound to the exosomes 32 bound to the magnetic microbeads 10 can be collected. Part (k) of FIG. 16B corresponds to the state in FIG. 12. The number of times the precipitates are collected and the supernatant is removed is not limited to the number of times illustrated in FIGS. 16A and 16B.

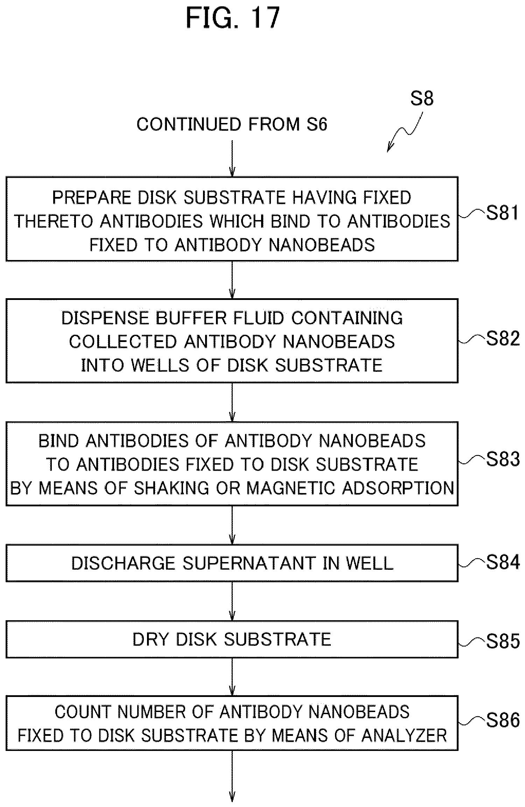

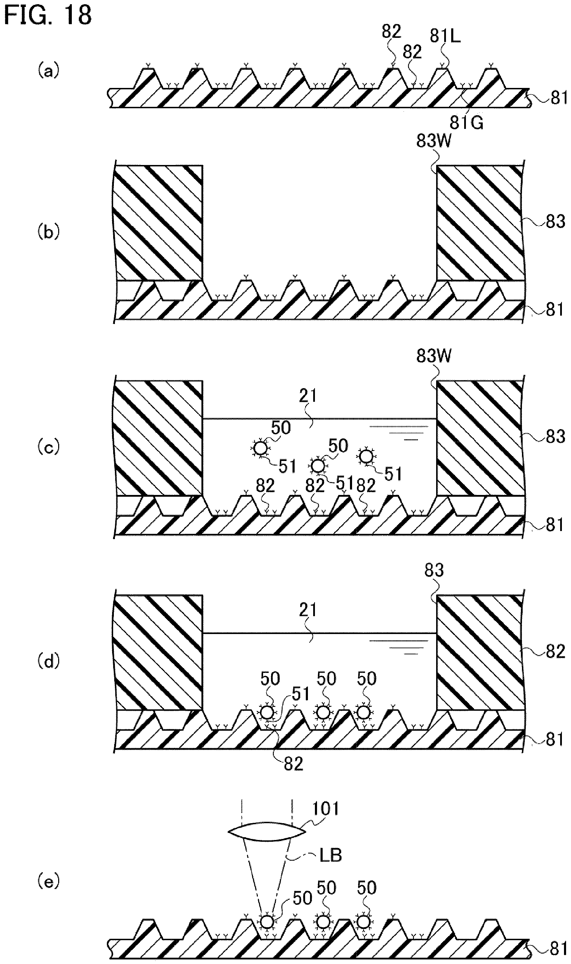

Referring to FIGS. 17 to 19, a description will be given regarding a specific method of counting the number of antibody nanobeads 50 in step S8 of FIG. 1. In FIG. 17, the operator, in step S81, prepares a disk substrate 81 having antibodies 82 fixed thereto, as illustrated in (a) of FIG. 18. The antibodies 82 bind to the antibodies 51 fixed to the antibody nanobeads 50. The antibody 82 is a goat anti-mouse IgG antibody, for example.

The disk substrate 81 is preferably disk-shaped, and recesses 81G and projections 81L are alternately arranged in the radial direction. The recesses 81G and the projections 81L are formed in a spiral or a concentric shape.

As illustrated in (b) of FIG. 18, the operator mounts a cartridge 83 having a plurality of, for example, cylindrical through-holes formed therein on the disk substrate 81. Thereafter, a well 83W is formed by the disk substrate 81 and the cartridge 83. As illustrated in (c) of FIG. 18, the operator dispenses the buffer fluid 21 containing the collected antibody nanobeads 50 into each well 83W in step S82. It should be noted that the cartridge 83 may have the configuration disclosed in Patent Literature 2.

As illustrated in (d) of FIG. 18, the operator binds the antibodies 51 of the antibody nanobeads 50 to the antibodies 82 fixed to the disk substrate 81 by means of shaking or magnetic adsorption at step S83. When a magnet is placed on the rear surface of the disk substrate 81 using the antibody nanobeads 50 as magnetic nanobeads, the antibody nanobeads 50 are attracted to the surface of the disk substrate 81. Thus, the antibodies 51 of the antibody nanobeads 50 are easily bound to the antibodies 82 fixed to the disk substrate 81.

The operator discharges the supernatant in the well 83W in step S84, and dries the disk substrate 81 in step S85. As illustrated in (e) of FIG. 18, in step S86, the operator mounts the dried disk substrate 81 to the analyzer, and counts the number of antibody nanobeads 50 fixed to the disk substrate 81 by means of the analyzer. The analyzer counts the number of antibody nanobeads 50 by irradiating the disk substrate 81 with a laser beam condensed by a condensing lens 101. It should be noted that the analyzer may have the configuration disclosed in Patent Literature 3.

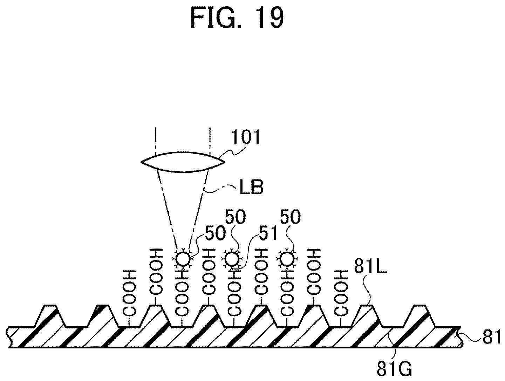

As illustrated in FIG. 19, instead of fixing the antibodies 82 to the disk substrate 81, the surface of the disk substrate 81 may be subjected to oxygen plasma treatment to form polar functional groups such as carboxyl groups (COOH). The antibodies 51 of the antibody nanobeads 50 are adsorbed to the polar functional groups.

As described above, in accordance with the biological sample analysis method according to one or more embodiments, using one biological sample (analyte 31), it is possible to analyze the inclusions of exosomes 32 containing a disease-associated antigen (membrane protein) and count the number of exosomes 32.

The present invention is not limited to the one or more embodiments described above, and may be varied in various ways without departing from the scope of the present invention. In one or more embodiments, the first bead is the magnetic microbead 10 and the second bead is the antibody nanobead 50, but the first bead and the second bead may be different in size so as to be separable from each other. When the first bead is a microbead and the second bead is a nanobead, they can be easily separated from each other. Thus, it is preferable that the first bead is a microbead and the second bead is a nanobead.

The first bead is preferably a magnetic bead but may be a non-magnetic bead. When the first bead is a magnetic bead and the second bead is a non-magnetic bead, these beads can be separated from each other very easily. When the second bead is a magnetic bead, the second bead can be easily fixed to the disk substrate 81. The first bead may be a non-magnetic bead, and the second bead may be a magnetic bead. Further, both the first bead and the second bead may be a magnetic bead.

This application claims priority under Japanese Patent Application No. 2020-024506 filed with the Japan Patent Office on February 17, 2020, the entire contents of all of which is incorporated herein by reference.

## Claims

1. A biological sample analysis method comprising:
injecting, into a reaction container, a first buffer fluid containing a plurality of a first bead having fixed to a surface thereof a first antibody that specifically binds to a first antigen associated with a particular disease;
injecting, into the reaction container, a plurality of a second bead having fixed to a surface thereof a second antibody that specifically binds to an optional second antigen that is the same as or different from the first antigen, and a biological sample containing an exosome to be analyzed having the first antigen and the second antigen on a surface of the exosome, thereby generating a second buffer fluid;
collecting the exosome to be analyzed having the first bead and the second bead bound thereto, from the second buffer fluid;
separating the collected exosome to be analyzed having the first bead and the second bead bound thereto, into the first bead, the second bead, and the exosome to be analyzed, and collecting the second bead and the exosome to be analyzed; and
analyzing an inclusion of the collected exosome to be analyzed, and counting the number of collected second beads.

2. The biological sample analysis method according to claim 1, wherein
the exosome to be analyzed having the first bead and the second bead bound thereto is dissolved to be separated into the first bead, the second bead, and an inclusion of the exosome to be analyzed, thereby collecting the second bead and the inclusion of the exosome to be analyzed.

3. The biological sample analysis method according to claim 1, wherein
the second bead and the exosome to be analyzed are bound to each other using a hapten,
the second bead is detached from the exosome to be analyzed by adding a hapten into a third buffer fluid containing the exosome to be analyzed having the first bead and the second bead bound thereto, thereby collecting the second bead, and
the exosome to be analyzed bound to the first bead is dissolved, thereby collecting an inclusion of the exosome to be analyzed.

4. The biological sample analysis method according to claim 1, wherein
the second antibody is fixed to the second bead by a cleavable linker,
the second bead is detached from the exosome to be analyzed by adding a cleavage reagent of the linker into a third buffer fluid containing the exosome to be analyzed having the first bead and the second bead bound thereto, thereby collecting the second bead, and
the exosome to be analyzed bound to the first bead is dissolved, thereby collecting an inclusion of the exosome to be analyzed.

5. The biological sample analysis method according to any one of claims 2 to 4, wherein a surfactant is used for the dissolving.

6. The biological sample analysis method according to any one of claims 1 to 5, wherein the first bead is a magnetic bead.
